(19) Europäisches Patentamt
European Patent Office
Office européen des brevets

(11) **EP 4 220 249 A1**

(12) **EUROPEAN PATENT APPLICATION**
published in accordance with Art. 153(4) EPC

(43) Date of publication:
**02.08.2023 Bulletin 2023/31**

(21) Application number: **21872122.3**

(22) Date of filing: **02.09.2021**

(51) International Patent Classification (IPC):
***G02B 5/26*** (2006.01)

(52) Cooperative Patent Classification (CPC):
**G02B 5/0247; A61L 2/10; G02B 5/0268;
G02B 5/0284**

(86) International application number:
**PCT/JP2021/032314**

(87) International publication number:
**WO 2022/064991 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.09.2020 JP 2020158811**

(71) Applicants:
• **Furukawa Electric Co., Ltd.**
 **Tokyo 100-8322 (JP)**
• **Daikin Industries, Ltd.**
 **Osaka-shi, Osaka 530-8323 (JP)**

(72) Inventors:
• **OKITA, Yutaka**
 **Tokyo 100-8322 (JP)**

• **TAKEMURA, Daiki**
 **Tokyo 100-8322 (JP)**
• **KOKUBO, Yousuke**
 **Tokyo 100-8322 (JP)**
• **HIROISHI, Jirou**
 **Tokyo 100-8322 (JP)**
• **ARASE, Takuya**
 **Osaka-shi, Osaka 530-8323 (JP)**
• **HIGUCHI, Tatsuya**
 **Osaka-shi, Osaka 530-8323 (JP)**
• **SUGIYAMA, Akinari**
 **Osaka-shi, Osaka 530-8323 (JP)**

(74) Representative: **Forstmeyer, Dietmar**
 **Boeters & Lieck**
 **Oberanger 32**
 **80331 München (DE)**

(54) **REFLECTIVE FILM MADE OF RESIN**

(57)    A resin reflective film, which containing two or more kinds of regions having different refractive indexes from each other, in which a film thickness of the resin film 20 to 5,000 μm, and a total reflectance is 60% or more and a diffuse reflectance is 60% or more for deep ultraviolet rays having a wavelength of 220 to 300 nm.

EP 4 220 249 A1

**Description**

FIELD OF THE INVENTION

The present invention relates to a resin reflective film.

BACKGROUND OF THE INVENTION

The sterilization effect of ultraviolet rays has been studied for a long time. As a light source of ultraviolet rays, a low-pressure mercury lamp, a xenon lamp, or the like has been the mainstream so far, but in recent years, an LED capable of emitting light having a wavelength in this ultraviolet region has been developed, and a sterilization equipment equipped with an LED or a sterilization method using an LED has been developed. For example, Patent Literature 1 describes a fluid sterilization module that irradiates a fluid flowing through a flow channel with ultraviolet rays to sterilize the fluid. In order to efficiently diffuse ultraviolet rays emitted from the light source to a certain region, it is effective to use a reflective material capable of reflecting ultraviolet rays efficiently and evenly. In the fluid sterilization module described in Patent Literature 1, an ultraviolet reflective material is used for an inner cylinder forming a cylindrical treatment-flow-path.

As an ultraviolet reflective material, a metal material, a resin material, and the like are known. As this metal material, for example, an aluminum foil for ultraviolet reflective materials that exhibits high reflectance for ultraviolet rays by controlling aluminum particles (Patent Literature 2), an aluminum reflective member having a reflective layer and a UV transmissive resin layer on the surface of an aluminum material, and the like are known (Patent Literature 3). As the resin material, a resin material in which fluororesins or silicone-based resins are formed into a multilayer laminate is known, and for example, a multilayer optical film having two fluoropolymer materials having different refractive indexes, and an ultraviolet reflective polymer film having two different polymer layers are known (Patent Literatures 4 and 5). In addition, a sintered and compressed or porous molded body made of polytetrafluoroethylene (PTFE) is also known as an ultraviolet reflective material.

CITATION LIST

PATENT LITERATURES

Patent Literature 1: JP-A-2019-187657 ("JP-A" means an unexamined published Japanese patent application)
Patent Literature 2: WO 2017/158989
Patent Literature 3: JP-A-2016-042183
Patent Literature 4: JP-A-7-507152
Patent Literature 5: JP-A-2015-165298

SUMMARY OF THE INVENTION

TECHNICAL PROBLEM

Commonly, a metal material specularly reflects (regularly reflects) ultraviolet rays. Therefore, for example, in the case of irradiating water or air with ultraviolet rays for sterilization, if a metal material is used as a reflective material, the reflection intensity (illuminance) of ultraviolet rays is weak depending on the angle even if the apparent reflectance is high, and ultraviolet rays cannot be evenly spread in the water or air, and it is difficult to obtain sufficient sterilization efficiency.

In addition, in a case where a multilayer laminate of fluororesins or silicone-based resins is used as a reflector, since the refractive index of a resin itself is limited, it is difficult to enhance the refractive index difference between the layers to a level at which sufficient reflectance is achieved. For this reason, there has been an existing situation where the reflection illuminance of deep ultraviolet is not sufficient in, for example, polymer films described in Patent Literatures 4 and 5.

Furthermore, a sintered and compressed or porous molded body made of PTFE has a large number of crystal grain boundaries or pores inside, and is excellent in ultraviolet ray reflection performance. However, in order to make this sintered and compressed porous molded body exhibit sufficient reflection performance for ultraviolet rays, it is necessary to secure a thickness of a certain level or more (for example, about 10 mm). As a result, in such a thick sintered and compressed porous molded body, the pliability is poor, the flexibility in processing is low, and the place where the ultraviolet reflective material is applied is restricted.

In view of the above circumstances, the present invention provides a resin reflective film having excellent diffuse reflection performance for ultraviolet rays, particularly for deep ultraviolet rays, excellent pliability, and high flexibility in processing.

SOLUTION TO PROBLEM

In the present invention, the above problems were solved by the following means:

(1) A resin reflective film, which contains two or more kinds of regions having different refractive indexes from each other,
wherein a thickness of the resin reflective film is 20 to 5,000 $\mu$m, and wherein a total reflectance is 60% or more and a diffuse reflectance is 60% or more for deep ultraviolet rays having a wavelength of 220 to 300 nm.
(2) The resin reflective film described in the above (1), wherein the thickness of the resin reflective film is 50 to 1,000 $\mu$m.
(3) The resin reflective film described in the above (1) or (2), wherein the two or more kinds of regions constituting the resin reflective film each have a light transmittance of 30 to 100% for deep ultraviolet rays having a wavelength of 220 to 300 nm.
(4) The resin reflective film described in the above (1) to (3), wherein at least one kind of the two or more kinds of regions constituting the resin reflective film is a bubble.
(5) The resin reflective film described in any of the above (1) to (4), containing a repeating structure portion in which a resin portion (resin region) and a void portion (gas region) repeat.
(6) The resin reflective film described in the above (5), wherein a width of at least one resin portion and/or a width of at least one void portion that constitute the repeating structure portion are 0.1 $\lambda$ to 20 A with respect to a wavelength $\lambda$ of incident ultraviolet rays.
(7) The resin reflective film described in any of the above (1) to (6), wherein a resin material constituting the resin reflective film is a fluorine-containing resin or a silicone resin; and
wherein the resin reflective film is obtained by allowing an inert gas impregnated into a film of the fluorine-containing resin or the silicone resin to effervesce.
(8) The resin reflective film described in any of the above (1) to (6), wherein a resin material constituting the resin reflective film is a fluorine-containing resin; and
wherein the resin reflective film is obtained by stretching a film of the fluorine-containing resin to generate a bubble and/or pore inside.
(9) The resin reflective film described in either of the above (7) or (8), wherein a density (Q) of the resin reflective film to a density (P) of the resin material constituting the resin reflective film satisfies $Q/P = 0.2$ to $0.99$.
(10) A sterilization device, containing:

an ultraviolet light source; and
the resin reflective film described in any of the above (1) to (9).

In general, "ultraviolet rays" refers to an electromagnetic wave having a wavelength shorter than that of visible light. In the present invention, the "deep ultraviolet rays" refers to an electromagnetic wave having a wavelength region of 200 to 300 nm.

In the present invention, the "total reflectance" means the sum of a "specular reflectance" and a "diffuse reflectance". The "specular reflectance" means a proportion of regularly reflected irradiation light to irradiation light, and the "diffuse reflectance" means a proportion of diffusely reflected irradiation light to irradiation light.

ADVANTAGEOUS EFFECTS OF INVENTION

The resin reflective film of the present invention is excellent in diffuse reflection performance for ultraviolet rays, particularly for deep ultraviolet rays, and is excellent in pliability and also has high flexibility in processing.

BRIEF DESCRIPTION OF DRAWINGS

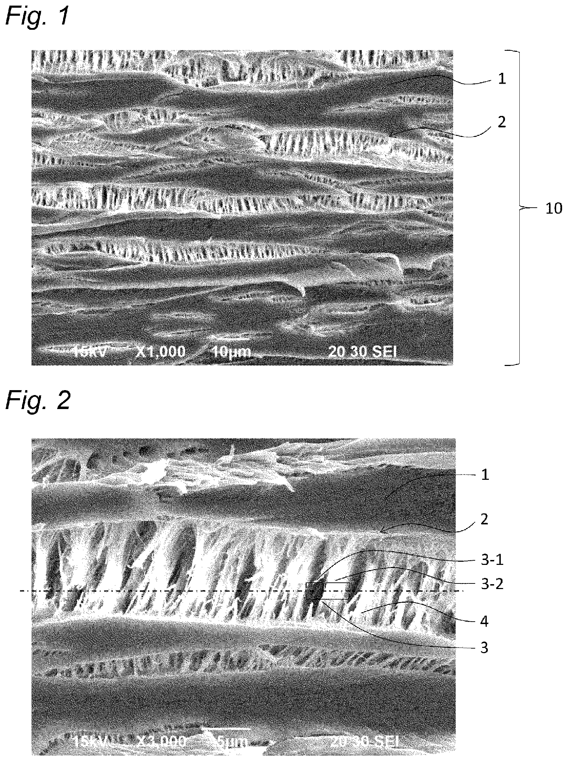

{Fig. 1}
Fig. 1 is a drawing-substituting photograph obtained by freeze-fracturing a reflective material produced in Example 1 in high vacuum and photographing a cross section thereof with a scanning electron microscope.

{Fig. 2}

Fig. 2 is a drawing-substituting photograph obtained by freeze-fracturing a reflective material produced in Example 1 in high vacuum and photographing a cross section thereof with a scanning electron microscope.

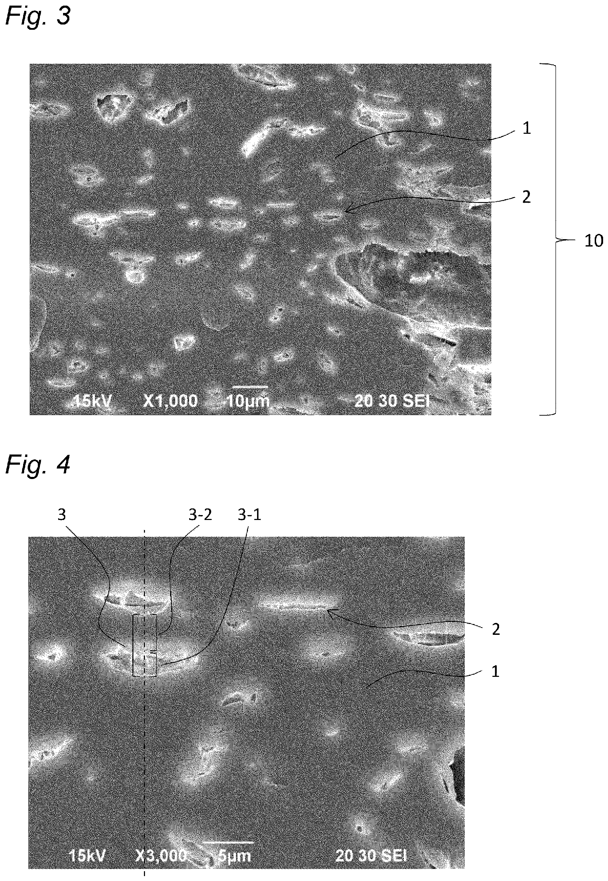

{Fig. 3}

Fig. 3 is a drawing-substituting photograph obtained by freeze-fracturing a reflective material produced in Example 6 in high vacuum and photographing a cross section thereof with a scanning electron microscope.

{Fig. 4}

Fig. 4 is a drawing-substituting photograph obtained by freeze-fracturing a reflective material produced in Example 6 in high vacuum and photographing a cross section thereof with a scanning electron microscope.

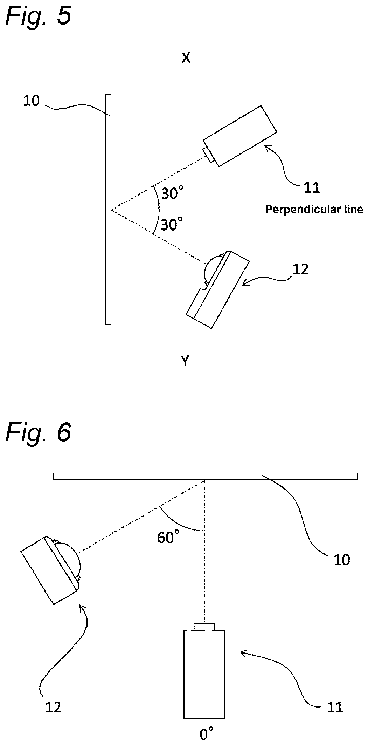

{Fig. 5}

Fig. 5 is a schematic diagram for describing a method of measuring ultraviolet illuminance in Test Example 2.

{Fig. 6}

Fig. 6 is a schematic diagram for describing a method of measuring ultraviolet illuminance in Test Example 2.

DESCRIPTION OF EMBODIMENTS

Preferable embodiments of the resin reflective film of the present invention will be described.

The resin reflective film of the present invention (hereinafter, also referred to as "reflective film of the present invention") has two or more kinds of regions having different refractive indexes from each other. The resin reflective film has such a structure and can thereby diffusely reflect deep ultraviolet rays efficiently as well as multidirectionally and uniformly. That is, the reflective film of the present invention has a total reflectance of 60% or more and a diffuse reflectance of 60% or more for deep ultraviolet rays having a wavelength of 220 to 300 nm. In addition, the thickness of the reflective film (film thickness) of the present invention is 20 to 5,000 $\mu$m.

The reflective film of the present invention exhibits desired sufficient reflection properties even in the form of a thin film. The film thickness of the reflective film of the present invention is preferably 30 $\mu$m or more, more preferably 40 $\mu$m or more, further preferably 50 $\mu$m or more, and also preferably 100 $\mu$m or more from the viewpoint of improving diffuse reflection performance for deep ultraviolet rays having a wavelength of 220 to 300 nm. In addition, the film thickness is preferably 3,000 $\mu$m or less, more preferably 2,000 $\mu$m or less, and further preferably 1,000 $\mu$m or less from the viewpoint of improving the pliability of the reflective film and increasing the flexibility in processing.

From the same viewpoint as described above, the film thickness of the reflective film of the present invention is preferably 30 to 3,000 $\mu$m, more preferably 40 to 2,000 $\mu$m, further preferably 50 to 1,000 $\mu$m, and even further preferably 100 to 1,000 $\mu$m.

The reflective film of the present invention preferably has a configuration in which regions having different refractive indexes from each other are alternately laminated from the viewpoint of enhancing the total reflectance and the diffuse reflectance for deep ultraviolet rays having a wavelength of 220 to 300 nm to a desired level. Such a laminated form also includes a configuration such that one region is present in a dotted or linear shape in another region in a cross-sectional observation. In addition, the reflective film may have a form such that the entire reflective film of the present invention has the above-described laminated configuration, or a part of the reflective film of the present invention has the laminated configuration.

In the present invention, regions having different refractive indexes from each other have different refractive indexes for deep ultraviolet rays having a wavelength of 220 to 300 nm between the respective regions. If the refractive indexes in the respective regions are different for a wavelength generally measured, such as visible light, the refractive indexes are commonly different also for deep ultraviolet rays having a wavelength of 220 to 300 nm. Note that, since the refractive index commonly becomes higher as the wavelength of irradiation light is shorter, "different refractive indexes for deep ultraviolet rays having a wavelength of 220 to 300 nm" means that the respective regions have different refractive indexes for the same wavelength. The difference in refractive index between the regions having different refractive indexes is preferably 0.005 or more, more preferably 0.01 or more, further preferably 0.05 or more, further preferably 0.1 or more, further preferably 0.2 or more, and further preferably 0.3 or more from the viewpoint of improving the diffuse reflectance of the reflective film. In addition, a realistic refractive index difference is 2.0 or less.

By increasing the refractive index difference between the regions having different refractive indexes, reflection at the interface between the regions having different refractive indexes increases, and as a result, the diffuse reflectance of the reflective film is improved.

The regions constituting the reflective film of the present invention each have a light transmittance for a deep ultraviolet rays having a wavelength of 220 to 300 nm of preferably 30% or more, more preferably 50% or more, and further preferably 60% or more in any region. In addition, the light transmittance is commonly 100% or less, and may be 95% or less. That is, components constituting the regions of the reflective film are preferably a substance or a gas having low absorption ability for deep ultraviolet rays. In the present invention, the "light transmittance" means light transmittance in a single region. That is, even when one region includes another region, the light transmittance in each single region is preferably 30% or more, more preferably 50% or more, and further preferably 60% or more. The ultraviolet ray reflection efficiency of a resulting reflective film can further be enhanced with such components. The light transmittance for deep ultraviolet rays having a wavelength of 220 to 300 nm can be measured by a method described in Examples mentioned later.

Among two or more kinds of regions having different refractive indexes from each other included in the reflective film of the present invention, one kind of region is a region including a resin. The resin may be a matrix. The resin used for the reflective film of the present invention includes a resin material having low absorption ability for deep ultraviolet rays having a wavelength of 220 to 300 nm. The ultraviolet ray reflection efficiency of a resulting reflective film to be obtained can further be enhanced by using such a resin material. In addition, two or more kinds of regions having different refractive indexes from each other may be regions each including a resin material having a different refractive index.

As the resin material, one kind or two or more kinds of resins selected from a fluorine-containing resin and a silicone resin are preferable. Among them, a fluorine-containing resin is more preferable from the viewpoint of reducing the rigidity and the influence on electronic components. As the fluorine-containing resin, one kind or two or more kinds of resins selected from polychlorotrifluoroethylene (PCTFE), a tetrafluoroethylene-ethylene copolymer (ETFE), a tetrafluoroethylene-perfluoroalkyl vinyl ether copolymer (PFA), polytetrafluoroethylene (PTFE), a tetrafluoroethylene-hexafluoropropylene copolymer (FEP), polyvinylidene fluoride (PVDF), and a tetrafluoroethylene/ perfluoroalkyl vinyl ether/chlorotrifluoroethylene copolymer (CPT) are preferable, and one kind or two or more kinds of resins selected from PCTFE, ETFE, PFA, and CPT are more preferable from the viewpoint of relatively easy melt processing and favorable mechanical properties. The fluorine-containing resins exemplified above each have a light transmittance of 30% or more for deep ultraviolet rays having a wavelength of 220 to 300 nm. Among them, it is preferable to use a resin having a light transmittance for deep ultraviolet rays having a wavelength of 220 to 300 nm of preferably 50% or more, and more preferably 60% or more.

These resin materials may include various additives such as a heat stabilizer, an organic lubricant, organic or inorganic fine particles, and an antistatic agent as long as the effects of the present invention are not impaired.

In addition, it is preferable that at least one kind of region among two or more kinds of regions having different refractive indexes from each other included in the reflective film of the present invention is a region including a gas, an inorganic material, or a liquid. From the viewpoint of further increasing the difference in refractive index between the regions having different refractive indexes, it is preferable that at least one kind of region among the regions having different refractive indexes is a region including a gas, that is, at least one kind of region is a bubble and/or pore in the reflective film of the present invention.

In the present invention, the "gas" refers to a gas present in a void inside a resin or an inorganic material, or at an interface therebetween, the void being formed as a bubble or pore. In addition, in the present invention, the "gas" is a concept including not only the atmosphere but also a gaseous body such as an inert gas that is deviated from the atmospheric composition. That is, it is preferable that the reflective film of the present invention has a bubble and/or pore inside, and can diffusely reflect deep ultraviolet rays efficiently as well as uniformly and multidirectionally by including this bubble or pore therein. The shape of the bubble and/or pore is not particularly limited, and is appropriately designed within a range in which the effect of the present invention is not impaired. For example, in planar view of the cross section, the shape may be a circular shape, an elliptical shape, a substantially elliptical shape such as an elongated elliptical shape, or a long elliptical shape having acute angles at both ends, the acute angles being formed by substantially circular arcs facing each other.

When the reflective film of the present invention has a region including an inorganic material, examples of the inorganic material include alumina, boron nitride, silica, and an alkaline earth metal fluoride.

When the reflective film of the present invention has a region including a liquid, examples of the liquid include water, an organosiloxane, and a fluorine-containing inert liquid.

When the regions included in the reflective film of the present invention include a region including a resin and a region including a gas, the reflective film of the present invention can be obtained by forming a film into a form having a bubble or pore inside the resin from the viewpoint of enhancing the total reflectance and the diffuse reflectance of a resulting film for deep ultraviolet rays having a wavelength of 220 to 300 nm to a desired level. That is, the form can be such that voids are interspersed in the resin material.

When the reflective film of the present invention has regions each including a resin having a different refractive index, each region includes a resin material having a different refractive index, and the form can also be such that the resin materials having different refractive indexes are laminated, or such that the regions including one resin material are interspersed in the region including another resin material, from the viewpoint of enhancing the total reflectance and the diffuse reflectance of a resulting film for deep ultraviolet rays having a wavelength of 220 to 300 nm to a desired level. Furthermore, a void such as bubble or pore can be formed in these regions including a resin or at the interface therebetween.

When the reflective film of the present invention has a region including a resin and a region including an inorganic material, a resin material and an inorganic material having a different refractive index from the resin material are used, and the form can also be such that the region including the inorganic material is interspersed in the region including the resin material from the viewpoint of enhancing the total reflectance and the diffuse reflectance of a resulting film for deep ultraviolet rays having a wavelength of 220 to 300 nm to a desired level. In addition, the form can also be such that, in the above region including the resin material, a region including a resin material having a different refractive index is further interspersed. Furthermore, a void such as bubble or pore may be formed in these regions including the resin material or inorganic material, or at the interface therebetween.

When the reflective film of the present invention includes a region including a resin and a region including a gas, the reflective film preferably has a form having a repeating structure portion in which a resin portion (resin region) and a void portion (gas region) repeat in a cross-sectional observation (planar view observation for the cross section) in order for the reflective film of the present invention to exhibit desired reflection performance for deep ultraviolet rays.

The reflective film of the present invention may have a thin resin column, a thin wall-like resin column, or a thin protrusion of the resin portion in the bubble and/or pore. When a large number of resin portions including the resin column, the wall-like resin column, the protrusion of the resin portion, or the like are formed in the same direction, the resin portion constituting the repeating structure portion refers to the resin column, and the void portion constituting the repeating structure portion refers to a space between the resin columns. That is, a plurality of resin columns may be formed in the same direction in the bubble and/or pore, and as a result, the resin portion and the void portion may have the repeating structure portion in which the resin portion and the void portion repeat. In the present invention, "the same direction" means substantially the same direction, and the present invention is not limited to a form of facing exactly the same direction as long as the effect of the present invention is not impaired. In the repeating structure portion, the width of at least one kind resin portion or the width (nm) of at least one kind void portion constituting the repeating structure portion is preferably 0.1 $\lambda$ to 20 $\lambda$, more preferably 0.2 $\lambda$ to 10 $\lambda$, and further preferably 0.5 $\lambda$ to 2 $\lambda$ in the direction in which the resin columns repeat, with respect to the wavelength $\lambda$ (nm) of incident ultraviolet rays. By setting the width (nm) within the above-described range with respect to the wavelength $\lambda$ (nm) of ultraviolet rays, the reflection performance in the reflective film can be enhanced.

Furthermore, the reflective film of the present invention may have a plurality of bubbles and/or pores having the above-described repeating structure portion. In the above-described cross section, it is preferable that two or more bubbles and/or pores are present, and more preferable that three or more bubbles and/or pores are present along the thickness of the reflective film.

Fig. 1 is a scanning electron micrograph of a cross section of an embodiment of the reflective film of the present invention cut along the thickness. Fig. 2 shows a repeating structure portion of Fig. 1 in a further enlarged manner. A reflective film (10) shown in Fig. 1 is a reflective film including a resin (1), which has bubbles (2) having an elongated, substantially elliptical shape in planar view inside, and in which a large number of thin columns (4) including the resin are formed inside the bubbles (2) along the minor axis as shown in Fig. 2. In the reflective film shown in Fig. 2, the entire inside of the bubble (2) having a substantially elliptical shape in planar view is a repeating structure portion (3) in which the above-described resin portion (3-2) and void portion (3-1) repeat. Along the thickness (along the longitudinal axis in the drawing) of the reflective film (10), a plurality of bubbles (2) having these repeating structure portions are present in an overlapping manner. Note that the form of the reflective film of the present invention is not limited to the form of Fig. 1 at all, and a target reflective film can be obtained by other methods, which is supported by Examples mentioned later.

For the reflective film of the present invention shown in Figs. 1 and 2, for example, an inert gas is impregnated into a resin film, and then a fine pore or bubble is formed inside by heating or the like, and thus, a target reflective film can be obtained.

In addition, the reflective film of the present invention has substantially circular or substantially elliptical bubbles in planar view of the cross section, in which these bubbles and/or pores are stacked along the thickness, and the reflective film may thus have a repeating structure portion in which the resin portion and the void portion repeat. This stacking may be either random or regular. In this case, the width of at least one kind resin portion and/or the width (nm) of at least one kind void portion constituting the repeating structure portion, which is preferably the width (nm) of at least one kind void portion constituting the repeating structure portion, is preferably 0.1 $\lambda$ to 20 $\lambda$, more preferably 0.2 $\lambda$ to 10 $\lambda$, and further preferably 0.5 $\lambda$ to 2 $\lambda$ with respect to the wavelength $\lambda$ (nm) of incident ultraviolet rays. In this case, the width of the void portion is the size of the bubble and/or pore, that is, the diameter of the bubble and/or pore, and the width of the resin portion is the interval between the bubbles and/or pores. Here, in the present invention, the "diameter of the bubble and/or pore" means the longest one of the widths perpendicular to the longest width of the bubble and/or pore in planar view of the film cross section. By setting the width (nm) within the above range with respect to the wavelength $\lambda$ (nm) of ultraviolet rays, the reflection performance in the reflective film of the present invention can be enhanced.

In planar view observation of the cross section, the diameter of the bubble and/or pore is also preferably controlled to 20 nm to 6,000 nm, can also be controlled to 40 nm to 3,000 nm, and can further be controlled to 100 nm to 1,000 nm. By setting the diameter of the bubble and/or pore within the above range, the reflection performance can be enhanced.

Furthermore, particles of a substance different from the resin serving as the matrix may be present inside the bubble and/or pore. The particles are preferably a material that absorbs less deep ultraviolet rays. In addition, the particles can be less likely to be mechanically deformed and thermally deformed than the resin constituting the matrix. Examples thereof include a fluororesin such as PTFE, boron nitride, alumina, glass frit, and silica (quartz). The particles may be derived from fine particles added when a pore (bubble) is formed by stretching as mentioned above and later.

In addition, the reflective film of the present invention may have elongated, substantially elliptical bubbles, which are stacked, and thus have a repeating structure portion in which the resin portion and the void portion repeat. The width (nm) of at least one kind of the resin portion or the void portion constituting the repeating structure portion may be in the above-mentioned preferable range for the repeating structure portion.

Fig. 3 is a scanning electron micrograph of a cross section of an embodiment of the reflective film of the present invention cut along the thickness. Fig. 4 shows a repeating structure portion of Fig. 3 in a further enlarged manner. A reflective film (10) shown in Fig. 3 is a reflective film including a resin (1), which has bubbles (2) having a substantially elliptical shape in planar view inside. In the reflective film shown in Fig. 4, a repeating structure portion (3) is formed, in which a void portion (3-1) including a bubble (2) having a substantially elliptical shape in planar view and a resin portion (3-2) including a resin (1) repeat. Along the thickness (longitudinal axis in the drawing) of the reflective film (10), a plurality of these repeating structure portions is present in an overlapping manner. Note that, in Fig. 4, although a leader line for a symbol 3-1 is drawn out from a bubble different from a bubble having a leader line for a symbol 2, this is to facilitate grasping of the repeating structure.

The form of the reflective film of the present invention is not limited to the form of Fig. 3 at all, and a target reflective film can be obtained by other forms, which is supported by Examples mentioned later.

Examples of a method of forming the reflective film of the present invention shown in Figs. 3 and 4 include a method in which organic or inorganic fine particles are added to a resin material, or organic or inorganic particles are added to a resin material together with a resin incompatible with the resin material, melt-extruded, and then stretched in at least one direction to form fine pores inside. In addition, it is also possible that a resin material is formed into a film, and then a physical force is applied to the film to generate fine cracks, and desired reflection properties is exhibited. It is also possible to obtain a target reflective film by adding foamable particles to a resin material and performing melt extrusion, or injecting an inert gas such as carbon dioxide gas or nitrogen into a resin material or a film formed article thereof and performing extrusion foaming.

In the reflective film of the present invention, the thickness (width) of the resin portion (resin wall) constituting the space between bubbles may be uniform or non-uniform, and may be different between the axis along the film plane and the axis along the film thickness. The thickness of the resin wall along the film plane may be thicker than the thickness of the resin wall along the film thickness. The thickness of the resin wall along the film plane is thicker than the thickness of the resin wall along the film thickness, which enables the reflective film to simultaneously have high reflectance, ease of bending of the film, and mechanical strength (tensile strength). That is, the thickness of the resin wall along the film thickness is thin, which enables a large number of repeating structures of the resin portion and the void portion to be imparted and enables the reflectance for ultraviolet rays to be enhanced. In addition, when the film is bent, this thin resin wall along the film thickness is deformed, which enables the film to be more easily bent and the pliability to be further imparted to the film. Meanwhile, the resin wall along the film plane is thick, which enables the film to have a high mechanical strength (tensile strength). For example, it is also preferable that the thickness of the resin wall along the film plane is 1 μm or more, and the thickness of the resin wall along the film thickness is less than 1 μm.

The cross-sectional observation can be performed using a scanning electron microscope.

The reflective film of the present invention has a total reflectance for deep ultraviolet rays of 220 to 300 nm is 60% or more as mentioned above, preferably 70% or more, more preferably 80% or more, and further preferably 90% or more. In the present invention, the "total reflectance for deep ultraviolet rays having a wavelength of 220 to 300 nm " means an average value of total reflectances at respective wavelengths (in 1 nm units, that is, every 1 nm) in the wavelength region of deep ultraviolet rays having a wavelength of 220 to 300 nm. The total reflectance for deep ultraviolet rays can be measured by a method described in Examples mentioned later.

The reflective film of the present invention also has a diffuse reflectance for deep ultraviolet rays of 220 to 300 nm of 60% or more as mentioned above. This diffuse reflectance is preferably 70% or more, more preferably 80% or more, and further preferably 89% or more. In the present invention, the "diffuse reflectance for deep ultraviolet rays having a wavelength of 220 to 300 nm" means an average value of diffuse reflectances at respective wavelengths (in 1 nm units, that is, every 1 nm) in the wavelength region of deep ultraviolet rays having a wavelength of 220 to 300 nm. The diffuse reflectance in the deep ultraviolet region having a wavelength of 220 to 300 nm can be measured by a method described in Examples mentioned later.

In the reflective film of the present invention, the density (bulk density, Q) of the film (film having a bubble or pore) is, with respect to the density (P) of a resin material itself constituting the film, preferably Q/P = 0.1 to 0.99, more preferably Q/P = 0.3 to 0.99, and further preferably Q/P = 0.5 to 0.99. Note that the unit of the density P and the unit of the density Q are the same. The density (bulk density) of the reflective film of the present invention can be measured by a water replacement method (JIS K 7112).

A method of producing the reflective film of the present invention will be described below.

<Preparation of reflective film by foaming fluorine-containing resin film>

The structure shown in Fig. 1 is from a film obtained by impregnating a PCTFE film with carbon dioxide gas and then heating and foaming the PCTFE film. By using a fluorine-containing resin as a resin material constituting the reflective film, it is possible to obtain a reflective film in which a large number of fine columnar structures are formed inside a bubble as shown in Fig. 1. An example of a method for obtaining a reflective film having such a specific bubble structure will be described.

The preparation method exemplified here includes a gas inclusion step of impregnating a fluorine-containing resin film with an inert gas (carbon dioxide gas, nitrogen, etc.) under high pressure, and a heating and foaming step of heating the fluorine-containing resin film after pressure release to generate a bubble inside the resin.

In the gas inclusion step, the fluorine-containing resin film is exposed to an inert gas under a pressure condition of preferably 1 to 20 MPa and more preferably 5 to 10 MPa, for preferably 1 to 100 hours and more preferably 2 to 24 hours, and the inert gas is thus included in the resin film. In this gas inclusion step, for example, an autoclave, a pressure pot, or the like can be suitably used.

In the heating and foaming step, the fluorine-containing resin film after the gas inclusion step is heated at a temperature condition of preferably 120 to 200°C and more preferably 130 to 170°C, for preferably 0.5 to 3 minutes and more preferably 0.5 to 1 minute. Through this step, a reflective film having a bubble or pore inside the resin film can be obtained.

Furthermore, it is preferable to subject the fluorine-containing resin film to a heat treatment (annealing treatment) before the above gas inclusion step. By subjecting to the annealing step and then shifting to the gas inclusion step, the inside of a bubble to be generated in the subsequent heating and foaming step can be allowed to have a finer columnar structure as shown in Fig. 1, and a repeating structure portion in which a resin portion and an air portion densely repeat can be introduced into the film. Therefore, the reflection efficiency for deep ultraviolet rays can effectively be enhanced, and the total reflectance for deep ultraviolet rays having a wavelength of 220 to 300 nm can be more reliably led to 60% or more, and the diffuse reflectance for deep ultraviolet rays having a wavelength of 220 to 300 nm can be more reliably led to 60% or more.

In the preparation method described above, the preparation of the reflective film by foaming the fluorine-containing resin film has been described. However, when another resin having low deep ultraviolet absorption ability such as a silicone resin is used, the reflective film of the present invention exhibiting target reflective performance can also be obtained by foaming in a similar manner.

In the reflective film obtained by the foaming, the size of the bubble formed inside the film (substantially elliptical bubble in Fig. 1) can be 0.1 to 50 μm, is more preferably 0.5 to 30 μm, and is also preferably 1 to 20 μm, as a size along the thickness in the cross-sectional observation.

<Preparation of reflective film by stretching treatment of fluorine-containing resin>

A structure shown in Fig. 3 is a film obtained by adding PTFE fine particles to a PCTFE film and then stretching the PCTFE film to generate voids therein. By using a fluorine-containing resin as a resin material constituting the reflective film, and adding a material having low absorption of deep ultraviolet rays as fine particles thereto to perform stretching, it is possible to obtain a reflective film in which a large number of fine pore structures (porous structures) are formed as shown in Fig. 3. An example of a method for obtaining a reflective film having such a porous structure will be described.

The production method exemplified here includes a stretching step of stretching a fluorine-containing resin film.

In the stretching step, the resin film is stretched at a low speed of, for example, about 0.05 to 1.5 m/min until the stress reaches the yield point of the resin film under a heated atmosphere (for example, 50 to 120°C), necking occurs after the yield point, and then the resin film is stretched at a higher speed of, for example, about 2.0 to 4.0 m/min. This stretching may be uniaxial stretching or biaxial stretching, and is preferably biaxial stretching from the viewpoint of increasing the number of resulting bubbles or pores.

Furthermore, when the resin film is subjected to the stretching treatment, it is preferable to add fine particles or the like as a component different from the resin in advance and to perform kneading by a melt-kneading method or the like. The resin contains fine particles or the like, which can thereby generate an interface between the resin film as a base material and the fine particles, and can generate fine bubbles or pores starting from the interface at the time of stretching.

Examples of the fine particles to be added include polytetrafluoroethylene (PTFE), boron nitride, alumina, and glass frit. The amount of the fine particles to be added is preferably 1 to 50 mass%, more preferably 1 to 30 mass%, and further preferably 5 to 20 mass%.

In the reflective film obtained by the above-described stretching, the size of the bubble formed inside the film (substantially elliptical bubble in Fig. 3) is preferably 0.1 λ to 20 λ, more preferably 0.2 λ to 10 λ, and further preferably 0.5 λ to 2 λ with respect to the wavelength λ (nm) of incident ultraviolet rays, as a size along the thickness in the cross-sectional observation. For example, the size of the bubble along the thickness in the cross-sectional observation can be 20 nm to 6,000 nm, is also preferably 40 to 3,000 nm, and is also preferably 100 to 2,000 nm.

Note that the resin reflective film of the present invention is practically difficult to express its minute and complicated structure accurately and unambiguously. Therefore, in the present invention, while the structural features are specified as the matters specifying the invention, the properties and, if necessary, the production method are also specified as the matters specifying the invention, and the invention is clarified by clearly indicating the difference from an object according to a conventional art.

The reflective film of the present invention having excellent total reflectance and diffuse reflectance can be used as, for example, a reflective film for a deep ultraviolet light source and thus efficiently reflect deep ultraviolet rays emitted from the light source. Therefore, for example, light deviated from an irradiation target is reflected for deep ultraviolet rays emitted from a mercury lamp, a metal halide lamp, a barrier discharge lamp, a deep ultraviolet LED, or the like, and deep ultraviolet rays can be used without fail. A unit in which such a light source and the reflective film are combined can be suitably used for water sterilization equipment, spatial sterilization equipment, equipment (sterilization devices) for sterilizing surfaces of substances such as medical supplies and daily necessities, various processed products and foods, and the like.

In addition, since the reflective film of the present invention highly reflects deep ultraviolet rays, thereby preventing the transmission thereof, it can also be used as, for example, a shielding film for protecting those exposed to deep ultraviolet rays.

EXAMPLES

The present invention will be described in more detail based on the following Examples and Comparative Examples, but the present invention is not limited thereto.

[Preparation of reflective film]

Reflective films of Examples 1 to 6 and Comparative Examples 1 to 3 were prepared by the following methods. The reflective films of Examples 1 to 6 and Comparative Examples 1 to 3 each had a length of 100 mm and a width of 33 mm and had a thickness as shown in the table below.

(Example 1)

A polychlorotrifluoroethylene (PCTFE) resin film (trade name: NEOFLON PCTFE, manufactured by Daikin Industries, Ltd.) was heat-treated under a 180°C atmosphere for 10 minutes. The resin film after the heat treatment was placed in an autoclave and treated under the conditions of 17°C and a pressure of 5.2 MPa for 24 hours, and carbon dioxide gas was included in the resin film. Thereafter, the resin film was taken out from the autoclave and heated at 150°C for 1 minute to allow the carbon dioxide gas to effervesce in the resin film, thus preparing a reflective film having a thickness of 0.2 mm.

(Example 2)

A reflective film was prepared in a similar manner to Example 1 except that the thickness of the resulting reflective film was 0.4 mm.

(Example 3)

A reflective film was prepared in a similar manner to Example 1 except that the thickness of the resulting reflective film was 0.8 mm.

(Example 4)

A reflective film was prepared in a similar manner to Example 1 except that the resin film used for preparing a reflective film was replaced with a film of a tetrafluoroethylene-ethylene (ETFE) copolymer (trade name: NEOFLON ETFE, manufactured by Daikin Industries, Ltd.).

(Example 5)

A reflective film was prepared in a similar manner to Example 1 except that the resin film used for preparing a reflective film was replaced with a film of a tetrafluoroethylene-perfluoroalkyl vinyl ether (PFA) copolymer (trade name: NEOFLON PFA, manufactured by Daikin Industries, Ltd.).

(Example 6)

PTFE particles (10 mass%) (trade name: POLYFLON PTFE, model number: M-12, particle diameter: 0.1 μm, manufactured by Daikin Industries, Ltd.) was added to the PCTFE resin, the composite material was formed into a film having a thickness of 0.5 mm, then mounted on a stretching machine (trade name: TENSILON Universal Testing Machine, model number: RTA-2.5T, manufactured by Orientec Corporation), and stretched under a 120°C atmosphere. The stretching was performed at a speed of 0.5 m/min until the yield point of the resin film was passed, the necking was started, and then the speed was shifted to 3.0 m/min without stretching interruption to continue the stretching, thus obtaining a reflective material having a thickness of 0.25 mm.

The reflective films of Examples 1 to 6 each had a repeating structure portion formed by repeating of a resin portion and a void portion, and the width of at least one resin portion and/or the width (nm) of at least one void portion that constitute the repeating structure portion was 0.1 λ to 20 λ with respect to the wavelength λ (256 nm) of incident ultraviolet rays.

The widths of the resin portion and the void portion were verified by freeze-fracturing each film in high vacuum, observing the cross section with a scanning electron microscope (model number: JSM-6390LV, manufactured by JEOL Ltd.), and measuring the width from the data obtained.

[Comparative Examples]

(Comparative Example 1)

A polyethylene terephthalate (PET) resin film (raw material trade name: UNIPET RT553C, manufactured by Japan Unipet Corporation) was heat-treated under a 180°C atmosphere for 10 minutes. The resin film after the heat treatment was placed in an autoclave and treated under the conditions of 17°C and a pressure of 5.2 MPa for 24 hours, and carbon dioxide gas was included in the resin film. Thereafter, the resin film was taken out from the autoclave and heated under a 220°C condition for 1 minute to allow the carbon dioxide gas in the resin film to effervesce, thus preparing a reflective film having a thickness of 0.5 mm.

(Comparative Example 2)

As a reflective film, an aluminum foil for ultraviolet ray reflection (trade name: MIRO-UV, manufactured by Material House Co., Ltd.) having a thickness of 0.5 mm was used.

(Comparative Example 3)

As a reflective film, a polytetrafluoroethylene plate (trade name: POLYFLON PTFE, model number: M-18, manufactured by Daikin Industries, Ltd., sintered and compression-molded body) having a thickness of 9.8 mm was used.

<Measurement of deep ultraviolet light transmittance of resin material>

Light beams having respective wavelengths were irradiated using a spectrophotometer (trade name: U-4100, manufactured by Hitachi High-Tech Corporation) toward the front of each film before heat treatment (before foaming) or before stretching (before forming pores), and the amount of light captured by a detector with respect to the amount of the irradiated light of 100% was taken as a transmittance, and the transmittance was measured over a deep ultraviolet region having a wavelength of 220 to 300 nm. Each transmittance at every 1 nm wavelength was read from the obtained chart (measurement result), and the arithmetic average of the transmittances for all wavelengths in the deep ultraviolet region (81 measured values (%)) was determined and taken as the transmittance of the deep ultraviolet rays. All the films for the measurement had a thickness of 100 μm.

<Test Example 1>

The thickness was measured with a micrometer (trade name: Coolant-proof micrometer, model number: MDC-25MX, manufactured by Mitutoyo Corporation) for each of the obtained reflective films (Examples 1 to 6 and Comparative Examples 1 to 3). A Φ 60 standard integrating sphere was attached to a spectrophotometer (trade name: U-4100, manufactured by Hitachi High-Tech Corporation), and the total reflectance of each reflective film with respect to the total reflectance value of a Spectralon standard reflector (manufactured by Labsphere Inc., white, model number: USRS-99-010) of 100% and the diffuse reflectance of each reflective film with respect to the diffuse reflectance of the Spectralon standard reflector of 100% were measured over a deep ultraviolet region having a wavelength of 220 to 300 nm. Each reflectance at every 1 nm wavelength was read from the obtained chart (measurement result), and the arithmetic average of the total reflectances (81 measurement values (%)) and the arithmetic average of the diffuse reflectances (81 measurement values (%)) in the deep ultraviolet region were determined and taken as the "deep ultraviolet total reflectance" and "deep ultraviolet diffuse reflectance", respectively. The results are shown in Table 1 below.

<Test Example 2>

The ultraviolet illuminance for each reflection angle was measured as follows for the obtained reflective films (Examples 1 to 6 and Comparative Examples 1 to 3) using an ultraviolet LED (emission wavelength: 256 nm, model number: 265-FL-02-G01, manufactured by DOWA Electronics Materials Co., Ltd.) and an ultraviolet illuminometer (trade name: UV Radiometer UVR-300, model number: UD-250, manufactured by Topcon Technohouse Corporation).

As shown in Fig. 5, the ultraviolet LED light source was located in a positional relationship of 30° with respect to the center (center of gravity) of the film surface of each reflective film (an angle between a straight line connecting the ultraviolet LED light source to the center of the film surface and a perpendicular line extending from the center of the film surface is 30°). In addition, the ultraviolet illuminometer was placed at a position line-symmetric to the ultraviolet LED light source with a perpendicular line extending from the center of the film surface as an axis. That is, a plane connecting the center of the film surface, the ultraviolet LED light source, and the ultraviolet illuminometer perpendicularly intersects the film surface, and an angle between a straight line connecting the ultraviolet illuminometer to the center of the film surface and the above-described perpendicular line is 30°. Both distances from the ultraviolet LED and the ultraviolet illuminometer to the center of the film surface were 40 mm.

In a state where the ultraviolet LED was fixed, the ultraviolet illuminometer was moved from a position of 0° to positions of 30° and 60° as shown in Fig. 6. Note that Fig. 6 is a schematic view of the reflective film, the ultraviolet LED light source, and the ultraviolet illuminometer illustrated in Fig. 5, as viewed from X to Y, and illustrates a state where the ultraviolet illuminometer has moved to a position of 60°. Each ultraviolet illuminance detected by the ultraviolet illuminometer at the position of 0°, 30°, or 60° was measured. The measurement results are shown in Table 1 below.

In addition, the retention of the ultraviolet illuminance when the angle of the illuminometer was moved from 0° to 30° or 60° was described as an "illuminance retention (%) " in Table 1 below. The illuminance retention (%) was determined by Formula 2 below. The "reflection" was evaluated as "o" for a case where the illuminance retentions were 50% or more at both angles of 30° and 60°, and as "×" for the other cases (less than 50%).

Illuminance retention (%) =

[ultraviolet illuminance at 30° or 60°]/[ultraviolet illuminance at 0°]

(Formula 2)

In Test Example 1 and Test Example 2, measurements of the thickness, the deep ultraviolet total reflectance, the deep ultraviolet diffuse reflectance, the ultraviolet illuminance, and the illuminance retention were performed at three random points in the surface of each reflective film (provided that a portion within 5 mm from the end is excluded). The values described in Table 1 below are average values of these three points.

<Test Example 3>

The bending workability of each of the obtained reflective materials (Examples 1 to 6 and Comparative Examples 1 to 3) was evaluated by the following evaluation method.

It was verified whether each reflective material could be bent to be placed along the inside of a resinous pipe having an inner diameter of 40 mm. The "bending workability" was evaluated as "o" for a case where a reflective material was placed by human power, and as "×" for a case where a reflective material could not be bent and not be placed by human power. The results are shown in Table 1 below.

[Table 1]

| | | Ex. 1 | Ex. 2 | Ex. 3 | Ex. 4 | Ex. 5 | Ex. 6 | CEx. 1 | CEx. 2 | CEx. 3 |
|---|---|---|---|---|---|---|---|---|---|---|
| Resin material used | | PCTFE | PCTFE | PCTFE | ETFE | PF A | PCTFE | PET | Aluminum foil | PTFE |
| Deep ultraviolet light transmittance of resin material (%) | | 93 | 93 | 93 | 86 | 60 | 93 | 0 | 0 | 21 |
| Thickness of reflective film | | 0.2 mm | 0.4 mm | 0.8 mm | 0.2 mm | 0.2 mm | 0.25 mm | 0.5 mm | 0.5 mm | 9.8 mm |
| Bubble, pore | | Exist | Exist | Exist | Exist | Exist | Exist | Exist | None | Exist |
| Q/P | | 0.76 | 0.72 | 0.68 | 0.85 | 0.86 | 0.95 | 0.33 | 1 | 1 |
| Deep ultraviolet total reflectance (%) | | 89.8 | 92.6 | 95.1 | 91.1 | 90.3 | 85.6 | 12.7 | 52.3 | 91.7 |
| Deep ultraviolet diffuse reflectance (%) | | 86.6 | 91.3 | 92.5 | 89.7 | 88.8 | 83.2 | 7.4 | 32.6 | 92.1 |
| Ultraviolet illuminance (pw/cm$_2$) | 0° | 38.2 | 43.3 | 46.5 | 42.6 | 38.4 | 36.4 | 0.5 | 80 | 40.3 |
| | 30° | 24.8 | 29.0 | 31.1 | 25.2 | 27.4 | 23.1 | 0.2 | 22 | 31.3 |
| | 60° | 21.1 | 25.2 | 27.1 | 22.2 | 23.3 | 20.0 | 0.1 | 6 | 19.7 |
| Illuminance retention (%) | 30° | 64.9 | 66.9 | 66.8 | 59.1 | 71.4 | 63.4 | 40.0 | 27.5 | 77.7 |
| | 60° | 55.2 | 58.2 | 58.3 | 52.1 | 60.8 | 54.9 | 20.0 | 7.5 | 48.9 |
| Evaluation | Bending workability | ○ | ○ | ○ | ○ | ○ | ○ | ○ | ○ | × |
| | Reflection retention | ○ | ○ | ○ | ○ | ○ | ○ | × | × | × |

Remarks: 'Ex.' means Example according to this invention, and 'CEx.' means Comparative Example.

From Table 1, in the reflective film of Comparative Example 1, which is a PET foam film, the result was that the PET absorbed deep ultraviolet rays, and the deep ultraviolet total reflectance and the deep ultraviolet diffuse reflectance were significantly low. In the reflective film of Comparative Example 2, which was an aluminum foil, the result was that the deep ultraviolet total reflectance and the deep ultraviolet diffuse reflectance were low, and the illuminance retention was also poor. The reflective film of Comparative Example 3, which is a sintered and compression-molded body of PTFE, has a favorable deep ultraviolet total reflectance and deep ultraviolet diffuse reflectance. However, there was angle dependence in the diffuse reflection, and the result was that the performance of diffusely reflecting the incident deep ultraviolet rays evenly in many directions was slightly poor. The reflective film of Comparative Example 3 had a large thickness of 9.8 mm, and was also poor in bending workability.

On the other hand, the reflective films of Examples 1 to 6 are films that had bubbles or pores generated inside the resin films and thereby achieved both a deep ultraviolet total reflectance and a deep ultraviolet diffuse reflectance of 80% or more though they are thin films. The resin reflective films exhibiting such reflection properties had low angle-dependence of diffuse reflection of deep ultraviolet rays, and was excellent in performance of diffusely reflecting incident deep ultraviolet rays evenly in many directions. In addition, it was also found that the films can be thinned, and the sufficient bending workability can be achieved.

The present invention has been described as related to the embodiments. It is our intention that the present invention not be limited by any of the details of the description unless otherwise specified, but rather be construed broadly within its spirit and scope as set out in the attached claims.

The present application claims priority of Patent Application No. 2020-158811, filed in Japan on September 23, 2020, which is herein incorporated by reference as part of the present specification.

DESCRIPTION OF SYMBOLS

1    Resin
2    Bubble
3    Repeating structure portion
3-1    Void portion
3-2    Resin portion
4    Column
10    Reflective film
11    Ultraviolet LED light source
12    Ultraviolet illuminometer

**Claims**

1.  A resin reflective film, which comprises two or more kinds of regions having different refractive indexes from each other,

    wherein a thickness of the resin reflective film is 20 to 5,000 $\mu$m, and
    wherein a total reflectance is 60% or more and a diffuse reflectance is 60% or more for deep ultraviolet rays having a wavelength of 220 to 300 nm.

2.  The resin reflective film according to claim 1, wherein the thickness of the resin reflective film is 50 to 1,000 $\mu$m.

3.  The resin reflective film according to claim 1 or 2, wherein the two or more kinds of regions constituting the resin reflective film each have a light transmittance of 30 to 100% for deep ultraviolet rays having a wavelength of 220 to 300 nm.

4.  The resin reflective film according to any one of claims 1 to 3, wherein at least one kind of the two or more kinds of regions constituting the resin reflective film comprises a bubble.

5.  The resin reflective film according to any one of claims 1 to 4, comprising a repeating structure portion in which a resin portion (resin region) and a void portion (gas region) repeat.

6. The resin reflective film according to claim 5, wherein a width of at least one resin portion and/or a width of at least one void portion that constitute the repeating structure portion are 0.1 λ to 20 λ with respect to a wavelength λ of incident ultraviolet rays.

7. The resin reflective film according to any one of claims 1 to 6,

   wherein a resin material constituting the resin reflective film is a fluorine-containing resin or a silicone resin; and
   wherein the resin reflective film is obtained by allowing an inert gas impregnated into a film of the fluorine-containing resin or the silicone resin to effervesce.

8. The resin reflective film according to any one of claims 1 to 6,

   wherein a resin material constituting the resin reflective film is a fluorine-containing resin; and
   wherein the resin reflective film is obtained by stretching a film of the fluorine-containing resin to generate a bubble and/or pore inside.

9. The resin reflective film according to claim 7 or 8, wherein a density (Q) of the resin reflective film to a density (P) of the resin material constituting the resin reflective film satisfies Q/P = 0.2 to 0.99.

10. A sterilization device, comprising:

    an ultraviolet light source; and
    the resin reflective film according to any one of claims 1 to 9.

Fig. 1

Fig. 2

Fig. 3

Fig. 4

Fig. 5

Fig. 6

## INTERNATIONAL SEARCH REPORT

<table>
<tr><td colspan="2">International application No.<br><br>**PCT/JP2021/032314**</td></tr>
</table>

### A. CLASSIFICATION OF SUBJECT MATTER

*G02B 5/26*(2006.01)i
FI: G02B5/26

According to International Patent Classification (IPC) or to both national classification and IPC

### B. FIELDS SEARCHED

Minimum documentation searched (classification system followed by classification symbols)

G02B5/26

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2019-176987 A (ASAHI KASEI CORP.) 17 October 2019 (2019-10-17) | 1-6, 9-10 |
| | claims, paragraph [0024], etc. | |
| Y | | 7-8 |
| Y | JP 2009-237435 A (TORAY IND., INC.) 15 October 2009 (2009-10-15) | 7-8 |
| | paragraph [0053] | |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **18 October 2021** | **02 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/032314**

| Patent document cited in search report | | | Publication date (day/month/year) | Patent family member(s) | Publication date (day/month/year) |
|---|---|---|---|---|---|
| JP | 2019-176987 | A | 17 October 2019 | US 2019/0298868 A1 claims, paragraph [0055], etc. CN 110316786 A | |
| JP | 2009-237435 | A | 15 October 2009 | (Family: none) | |

Form PCT/ISA/210 (patent family annex) (January 2015)

**REFERENCES CITED IN THE DESCRIPTION**

**Patent documents cited in the description**

- JP 2019187657 A
- WO 2017158989 A
- JP 2016042183 A

- JP 7507152 A
- JP 2015165298 A
- JP 2020158811 A